# EUROPEAN PATENT APPLICATION

(11) **EP 4 793 955 A1**
(43) Date of publication of application: **19.08.2026**
(21) Application number: 24923594.6
(22) Date of filing: 31.12.2024
(51) Int. Cl.: G16C 20/30, G06N 3/04, G06N 3/08

(54) **METHOD AND APPARATUS FOR PREDICTING CHEMICAL FORM AND CHEMICAL REACTION OF MULTIPHASE SYSTEM**

(30) Priority: 06.02.2024 CN 202410171205
(71) Applicant: Tsinghua University, Beijing 100084 (CN)
(72) Inventor: XIE, Feng, Beijing 100084 (CN); WANG, Yu, Beijing 100084 (CN); GUO, Jingni, Beijing 100084 (CN); CAO, Jianzhu, Beijing 100084 (CN)
(74) Representative: Boult Wade Tennant LLP
(86) International application number: PCT/CN2024/144084
(87) International publication number: WO 2025/167392

(57) **Abstract**

This application provides a method and apparatus for predicting chemical states and a chemical reaction in a multiphase system. The method includes: obtaining a key condition corresponding to the multiphase system, and the key condition includes: temperature, pressure, a plurality of element types, and respective amounts thereof; predicting and obtaining chemical state information under the key condition by using a Gibbs free energy minimization method, and the chemical state information includes a plurality of pure substance species and respective amounts thereof when the multiphase system reaches thermodynamic equilibrium; predicting and obtaining a chemical reaction that will occur in the multiphase system under the key condition based on the chemical state information. This application can improve the accuracy and efficiency of predicting the chemical reaction in the multiphase system, based on the prediction of the chemical states of elements in the system.

## Description

### TECHNICAL FIELD

The present application relates to the technical field of multiphase system in chemical engineering, energy, and environmental sciences, particularly to methods and apparatus for predicting chemical states and chemical reaction in multiphase system.

### BACKGROUND

The chemical state of the element significantly impacts its physical and chemical properties, including melting point, volatility, adsorption-desorption behavior, diffusion process, and chemical reaction. Particularly, the interaction between elements and materials-such as adsorption, desorption, diffusion, deposition, and corrosion-are strongly dependent on the chemical state of the element. Therefore, the study of chemical state and chemical reaction of element is crucial in various fields, including nuclear energy system, chemical engineering, geochemistry, and metallurgy. This research underpins advancements in material design, energy utilization, environmental protection, and other related areas.

Currently, research on the chemical state of element in the multiphase system primarily focuses on thermodynamic equilibrium calculations to determine the stable chemical state within the system. Some studies have used a thermodynamic calculation method to investigate the complex chemical reaction in nuclear fuels of water-cooled reactor and to predict the chemical state of fission product under different oxygen potentials and different temperatures during an accident. Taking the Cs-Sr-Ag-I-O five-component system in the primary loop of a High-temperature Gas-cooled Reactor Pebble-bed Module (HTR-PM) under an equilibrium core condition as an example, existing research has enabled the calculation of the chemical state of some certain fission products under both a normal operating condition and an accident scenario.

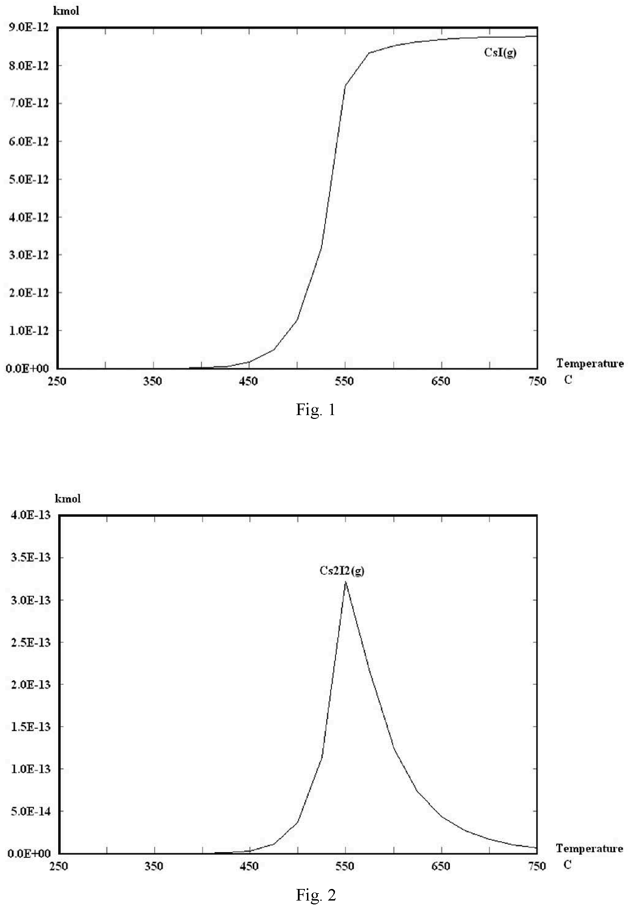

However, as shown in Figures 1 and 2, existing methods are merely limited to a software-generated result, and the change in the amount of respective element in the multiphase system with temperature is analyzed and obtained through the software-generated result. Conventional two-dimensional phase diagram can describe the system with relatively simple elemental compositions, but when a system contains a large number of elements, existing phase diagrams cannot provide important information for all elements. Up to now, no study has been reported on screening a potential chemical reaction according to the chemical states of elements, and the chemical process involved remain unclear. Furthermore, no specific method has yet been proposed for simultaneously predicting the chemical state of element and the corresponding chemical reaction thereof.

### SUMMARY

To address at least one problem in the prior art, the present application proposes a method and an apparatus for predicting the chemical states and the chemical reaction in the multiphase system. This disclosure not only enables the prediction of the chemical states of elements within the multiphase system, but also improves the accuracy and efficiency of predicting the chemical reaction occurring within the system.

To solve the above technical problems, the present application provides the following technical solution:
In a first aspect, the present application provides a method for predicting the chemical states and a chemical reaction in a multiphase system, including:
obtaining a key condition corresponding to the multiphase system, wherein the key condition comprises: temperature, pressure, a plurality of element types, and respective amounts thereof;
predicting and obtaining chemical state information under the key condition by using a Gibbs free energy minimization method; and the chemical state information comprises a plurality of pure substance species and respective amounts thereof when the multiphase system reaches thermodynamic equilibrium;
predicting and obtaining a chemical reaction that will occur in the multiphase system under the key condition based on the chemical state information.

In one embodiment, a plurality of key conditions are provided, a key influencing parameter of the multiphase system include at least one selected from a impurity element type among the plurality of element types, temperature, and pressure, values of the key influencing parameter corresponding to each key condition are different,
correspondingly, after acquiring the key conditions corresponding to the multiphase system, the method further includes:
predicting and obtaining the chemical state information under each key condition by using a Gibbs free energy minimization method;
predicting and obtaining the chemical reaction that will occur in the multiphase system under each key condition based on the chemical state information under each key condition.

In one embodiment, after predicting and obtaining the chemical state information under each key condition using the Gibbs free energy minimization method, the method further includes:
selecting, from the chemical state information under each key condition, the pure substance species containing a preset selected element and respective amounts thereof;
generating a two-dimensional projected phase diagram corresponding to the preset selected element according to the pure substance species containing the preset selected element and respective amounts thereof;
and the two-dimensional projected phase diagram is divided into a plurality of regions, each region corresponds to a group of pure substances, and the groups of the pure substances corresponding to the respective regions are different, each group of the pure substances is obtained according to the pure substance species containing the preset selected element, a value of a phase boundary line between the respective regions is a preset phase boundary line value, the two-dimensional projected phase diagram further includes: variation curves of each pure substance species containing the selected element.

In one embodiment, selecting, from the chemical state information under each key condition, the pure substance species containing the preset selected element and the respective amounts thereof includes:
performing interpolation processing based on the respective key conditions and chemical state information corresponding thereto to obtain interpolated key conditions and chemical state information corresponding respectively thereto;
selecting, from the chemical state information under each interpolated key condition, the pure substance species containing the preset selected element and the respective amounts thereof.

In a second aspect, the present application provides an apparatus for predicting chemical states and a chemical reaction in multiphase systems, including:
obtainment module configured to obtain a key condition corresponding to the multiphase system, and the key condition include: temperature, pressure, a plurality of element types, and respective amounts thereof;
first calculation module configured to predict and obtain chemical state information under the key condition by using a Gibbs free energy minimization method, and the chemical state information comprises a plurality of pure substance species and respective amounts thereof when the multiphase system reaches thermodynamic equilibrium;
first prediction module configured to predict and obtain a chemical reaction that will occur in the multiphase system under the key condition based on the chemical state information.

In one embodiment, a plurality of key conditions are provided, a key influencing parameter of the multiphase system include at least one selected from a impurity element type among the plurality of element types, temperature, and pressure, values of the key influencing parameter corresponding to each key condition are different,
correspondingly, the apparatus for predicting the chemical states and the chemical reaction in the multiphase system further includes:
second calculation module configured to predict and obtain the chemical state information under each key condition by using a Gibbs free energy minimization method;
second prediction module configured to predict and obtain the chemical reaction that will occur in the multiphase system under each key condition based on the chemical state information under each key condition.

In one embodiment, the apparatus for predicting the chemical states and the chemical reaction in the multiphase system further includes:
extraction module configured to select, from the chemical state information under each key condition, pure substance species containing a preset selected element and respective amounts thereof;
phase diagram plotting module configured to generate a two-dimensional projected phase diagram corresponding to the preset selected element according to the pure substance species containing the preset selected element and the respective amounts thereof;
and the two-dimensional projected phase diagram is divided into a plurality of regions, each region corresponds to a group of pure substances, and the groups of the pure substances corresponding to the respective regions are different, each group of the pure substances is obtained according to the pure substance species containing the preset selected element, a value of a phase boundary line between the respective regions is a preset phase boundary line value, the two-dimensional projected phase diagram also includes: the variation curves for each of the pure substances containing the selected elements.

In one embodiment, the extraction module includes:
interpolation processing unit configured to perform interpolation processing based on the respective key conditions and chemical state information corresponding thereto to obtain the interpolated key conditions and chemical state information corresponding respectively thereto;
extraction unit configured to select, from the chemical state information under each interpolated key condition, the pure substance species containing the preset selected element and the respective amounts thereof.

In a third aspect, the present application provides an electronic device, including a memory, a processor, and a computer program stored in the memory and executable by the processor, and the processor, when executing the program, implements the method for predicting the chemical states and the chemical reaction in the multiphase system.

In a fourth aspect, the present application provides a computer-readable storage medium, storing a computer instruction, and when executed by a processor, the instruction implement the method for predicting the chemical states and the chemical reaction in the multiphase system.

From the above technical solutions, it can be seen that the present application provides the method and the apparatus for predicting the chemical states and the chemical reaction in the multiphase systems. Specifically, the method includes: obtaining the key condition corresponding to the multiphase system, and the key condition include: temperature, pressure, a plurality of types of elements, and respective concentrations thereof; predicting and obtaining the chemical state information under the given key condition by using the Gibbs free energy minimization method, and the chemical state information includes a plurality of pure substance species and respective concentrations thereof when the multiphase system reaches thermodynamic equilibrium; and predicting and obtaining the chemical reaction that will occur in the multiphase system under the key condition based on the chemical state information. This method improves the accuracy and efficiency of predicting chemical reactions in multiphase systems based on the predicted chemical states of elements within the system. Specifically, the method not only provides information on the chemical states composition in the phase diagram but also simultaneously offers the variation of the relative concentrations of each chemical state along the X-axis. This makes the phase diagram information richer and more conducive to subsequent analysis, allowing for a clearer description of the composition of chemical states in the multiphase system and a clear display of the trends in the changes of the chemical states and concentrations of each element.

### BRIEF DESCRIPTION OF THE DRAWINGS

In order to more clearly illustrate the technical solutions of the embodiments of the present application or the prior art, a brief introduction to the drawings that will be used in the description of the embodiments or prior art is provided below. It is obvious that the drawings described below are merely some embodiments of the present application. For ordinary technical personnel in this field, other drawings can be obtained according to these drawings without paying any inventive labor.
**FIG. 1** is a schematic diagram illustrating a curve of CsI varying with temperature in a multiphase system according to an example of the prior art;
**FIG. 2** is a schematic diagram illustrating a curve of Cs₂I₂ varying with temperature in a multiphase system according to an example of the prior art;
**FIG. 3** is a logical schematic diagram of a method for predicting chemical states and a chemical reaction in a multiphase system according to an embodiment of the present application;
**FIG. 4** is a first flowchart of the method for predicting chemical states and a chemical reaction in a multiphase system according to an embodiment of the present application.
**FIG. 5** is a second flowchart of the method for predicting chemical states and a chemical reaction in a multiphase system according to an embodiment of the present application;
**FIG. 6** is a third flowchart of the method for predicting chemical states and a chemical reaction in a multiphase system according to an embodiment of the present application;
**FIG. 7** is a two-dimensional projected phase diagram of iodine (I) in the O-T system according to an embodiment of the present application.
**FIG. 8** is a fourth flowchart of the method for predicting chemical states and a chemical reaction in a multiphase system according to an embodiment of the present application;
**FIG. 9** is a schematic diagram illustrating the distribution of the Gibbs free energy of chemical reactions of iodine (I) in a multiphase system according to an embodiment of the present application;
**FIG. 10** is a schematic diagram of the structure of the apparatus for predicting chemical states and a chemical reaction in a multiphase system according to an embodiment of the present application;
**FIG. 11** is a schematic block diagram of the system configuration of the electronic device according to an embodiment of the present application.

### DESCRIPTION OF THE EMBODIMENTS

In order to enable those skilled in the art to better understand the technical solutions set forth in the present specification, the technical solutions in the embodiments of the present application will be clearly and completely described below with reference to the drawings of the embodiments of the present application. Obviously, the described embodiments are only some, rather than all, of the embodiments of the present application. All other embodiments obtained by a person of ordinary skill in the art based on the embodiments of the present application without any inventive efforts shall fall within the protection scope of the present application.

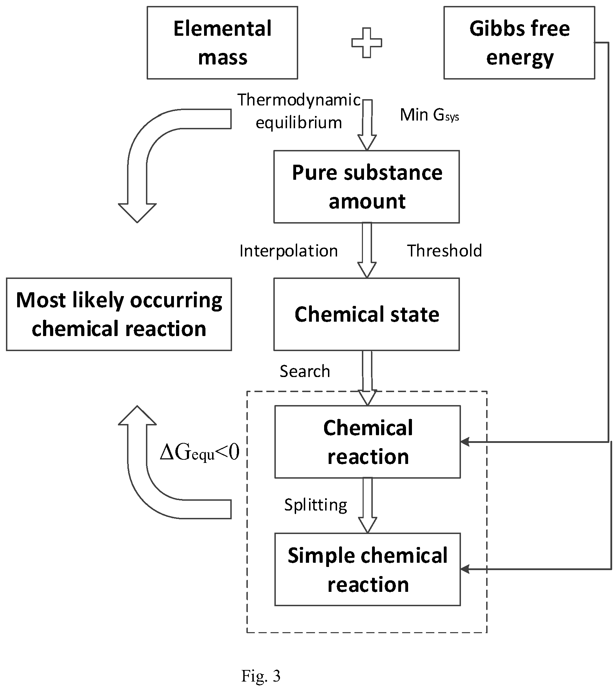

In order to solve the above problems existing in the prior art, an embodiment of the present application provides a method and apparatus for detecting chemical reaction in a multiphase system, as shown in Figure 3. According to the elemental amounts and chemical environment of the multiphase system, the amounts of pure substances under thermodynamic equilibrium can be calculated by minimizing Gibbs free energy. By analyzing the data, the chemical states of the elements under given conditions can then be obtained. Finally, based on the analyzed chemical states, possible chemical reactions that may occur in the process are searched and decomposed into simple chemical reactions. In combination with the criterion that the Gibbs free energy change is less than 0 and the thermodynamic equilibrium conditions, the chemical reactions most likely to occur in the multiphase system are ultimately determined. Specifically, a method may be provided for batch processing a multiphase system based on a thermodynamic calculation method or software, and for studying and analyzing the chemical states and chemical reactions obtained from such processing. The method may be used to analyze the chemical states of the multiphase system and to summarize and analyze possible chemical reactions occurring therein. By invoking the equilibrium calculation module of FactSage or other thermodynamic software, thermodynamic equilibrium calculations can be completed in batch. A two-dimensional projected phase diagram of the system can be plotted, and the error of the phase diagram can be discussed when other parameters are fixed. Complex reactions and simple reactions that may occur in the multiphase system can be determined. The effects of changes in key influencing parameters on the chemical states and chemical reactions of the elements are discussed. In the present solution, the chemical state information of elements in the multiphase system is obtained through thermodynamic calculations, and on the basis of the calculation of the chemical states of the elements in the multiphase system, possible chemical reactions in the multiphase system can be predicted in combination with the Gibbs free energy criterion. This study deepens the understanding of chemical processes in multiphase systems, provides an important foundation for investigating the reaction mechanisms and chemical behavior of elements in multiphase systems, and enables this method to be widely applied in industrial scenarios such as chemical engineering, energy, and environmental protection. Taking a nuclear energy system as an example, when interactions between elements and materials (such as adsorption, desorption, and deposition) are to be studied, the adsorption, deposition, and transport behavior of an element on piping may differ significantly depending on the chemical state in which the element exists. After the chemical states of the elements are determined by means of the present solution, key issues may be investigated in a targeted manner, trial-and-error costs may be reduced, and the physicochemical behavior of the elements may be described more accurately. In addition, in nuclear energy systems, effluents need to be monitored and controlled. Once the chemical states present in the system are determined, measurement methods, monitoring systems, and control measures can be designed in a targeted manner, thereby improving the accuracy and efficiency of monitoring in nuclear power plants.

The details are described below with reference to the following embodiments.

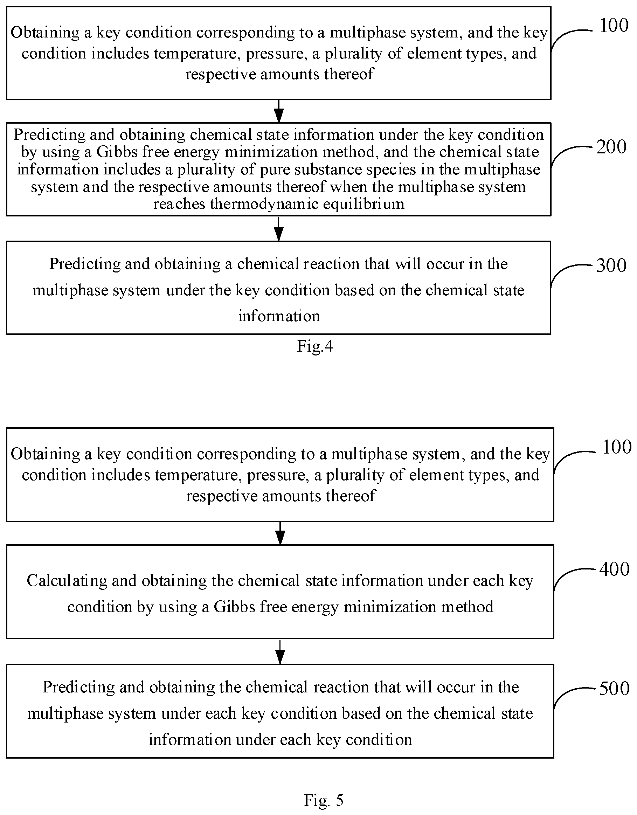

In order to improve the accuracy and efficiency of predicting a chemical reaction in a multiphase system on the basis of predicting the chemical states of elements in the multiphase system, the present embodiment provides a method for predicting the chemical states and a chemical reaction in a multiphase system, executed by a prediction apparatus for the chemical states and the chemical reaction in the multiphase system. The prediction apparatus for the chemical states and the chemical reaction in the multiphase system includes, but is not limited to, a server. As shown in Figure 4, the method specifically includes the following:

Step 100: obtaining a key condition corresponding to a multiphase system, and the key condition includes temperature, pressure, a plurality of element types, and respective amounts thereof.

Specifically, the key condition may be obtained from experimental measurements and theoretical calculations. In one example, the temperature is 2.00E+02 (°C), the pressure is 5.00E-03 (GPa), and the plurality of element types corresponding to the multiphase system and the respective amounts thereof are as follows:
(6.18E-08)Kr+(3.82E-07)Xe+(2.58E-10)I+(6.40E-13)Sr+(5.96E-10)Cs+(6.73E-12)Ag+(1.75E-11)Co+(3.88E-13)Fe+(3.73E-15)Cr+(1.81E-15)Mn+(3.33E-13)Ni+(7.93E-12)Rb+ (750000)He+(30.75)C+(4.99)H+(6.11)O+(0.20)N, the unit of amount is mole (mol).

Step 200: predicting and obtaining chemical state information under the key condition by using a Gibbs free energy minimization method, and the chemical state information includes a plurality of pure substance species in the multiphase system and the respective amounts thereof when the multiphase system reaches thermodynamic equilibrium.

Specifically, the chemical states of elements in the multiphase system may be represented by the elemental substances and compounds containing the element in the multiphase system.

For example, the chemical state information under the key condition includes pure substance species such as H, H₂, He, C, and C₂, and the amounts of H, H₂, He, C, and C₂ are (2.88E-20) mol, (4.52E-01) mol, (7.50E+05) mol, (1.17E-67) mol, and (0.00E+00) mol, respectively. The plurality of pure substances may be composed of the plurality of elements described above. The pure substance species may include an elemental substance and a compound.

Step 300: predicting and obtaining a chemical reaction that will occur in the multiphase system under the key condition based on the chemical state information.

Specifically, based on the chemical state information, the chemical reaction to occur in the multiphase system under the key condition can be predicted by means of a chemical equation balancing method. Among the plurality of pure substance species, target pure substance species containing a preset selected element can be selected. Based on the target pure substance species and the amounts thereof, the chemical reaction related to the preset selected element and occurring in the multiphase system under the key condition can be predicted by means of the chemical equation balancing method. Further, a simple chemical equation may be screened out from the predicted chemical reaction, Gibbs free energy values of the respective pure substances in the simple chemical equation can be obtained and substituted into the simple chemical equation to obtain the Gibbs free energy change of the corresponding simple chemical reaction, and if the Gibbs free energy change of the simple chemical reaction is less than zero, the simple chemical reaction is determined to occur spontaneously. The preset selected element may be at least one selected form a plurality of elements, such as iodine (I).

For example, all elements related to iodine (I) may first be searched and screened out by setting the element condition. For instance, when the element condition is set as I, O, Rb, and H, the pure substance species related to iodine obtained after the searching and screening include HIO, IRb, and the like. By using a chemical equation balancing algorithm, the chemical reactions can be screened out to include: 1I(g)+1Rb(g)-1IRb(g), 2HIO (g)+1H₂(g)→2I(g)+2H₂O(g), and so on.

Specifically, by applying a chemical equation balancing algorithm, balanced chemical equations for pure substance species related to the element I can be obtained. Then, according to a chemical equation combination and decomposition algorithm, the simple chemical equation in the system can be obtained from the chemical equations for the pure substance species related to the element I, and the total number of reactants and products is less than or equal to four, and in most cases there are only one or two reactants. For the simple chemical equation, Gibbs free energy values in the g0table are substituted into the simple chemical equation (i.e., the screened equations) to calculate the Gibbs free energy change of the simple chemical equation. If the Gibbs free energy change of the chemical reaction is less than zero, the chemical reaction is determined to be capable of occurring spontaneously. The process of applying the chemical equation balancing algorithm and the combination and decomposition algorithm is specifically as follows:

The chemical equation may be written as: ${n}_{{r}_{1}} {R}_{1} + ⋯ + {n}_{{r}_{{n}_{r}}} {R}_{{n}_{r}} = {n}_{{p}_{1}} {P}_{1} + ⋯ + {n}_{{p}_{{n}_{p}}} {P}_{{n}_{p}}$

Where:
*Rⱼ* and *Pⱼ* respectively represent the components of reactant *j* and product *j*; the reactants and products can be pure substances corresponding to the pure substance species in the above chemical state information.

*nᵣⱼ* and *nₚⱼ* respectively represent the stoichiometric coefficients of reactant *j* and product *j*.

In the chemical equation, the elemental system components on both sides of the equation sign must be identical: ${AN}_{r} = {BN}_{p}$ ${\sum }_{j}^{{n}_{r}} {a}_{ij} {n}_{{r}_{j}} = {\sum }_{j}^{{n}_{p}} {b}_{ij} {n}_{{p}_{j}}$ Where: $A = \left(\begin{matrix}{a}_{11} & … & {a}_{1 {n}_{r}} \\ … & … & … \\ {a}_{m 1} & … & {a}_{{mn}_{r}}\end{matrix}\right)$ $B = \left(\begin{matrix}{b}_{11} & … & {b}_{1 {n}_{p}} \\ … & … & … \\ {b}_{m 1} & … & {b}_{{mn}_{p}}\end{matrix}\right)$ ${N}_{r} = {\left({n}_{{r}_{1}},…,{n}_{{p}_{{n}_{r}}}\right)}^{T}$ ${N}_{p} = {\left({n}_{{p}_{1}},…,{n}_{{p}_{{n}_{p}}}\right)}^{T}$

In the above equation, *m* denotes the number of system components (elements) in the multiphase system, *nᵣ* denotes the number of reactants, and *nₚ* denotes the number of products. *aᵢⱼ* and *bᵢⱼ* denote the proportions of system component *i* corresponding to component *j* in the reactants and products, respectively. *Nᵣ* and *Nₚ* are vector expressions of the stoichiometric coefficients of the reactants and products.

The above equation may be normalized as: ${CN}_{e} = 0$ ${\sum }_{j}^{{n}_{e}} {c}_{ij} {n}_{{e}_{j}} = 0$ Where: $C = \left(A,-B\right)$ ${N}_{e} = {\left({N}_{r}, {N}_{p}\right)}^{T}$ ${C}_{ij} = \left\{\begin{matrix}{a}_{ij} & j = 1 to {n}_{r} \\ {b}_{i \left(j-{n}_{r}\right)} & j = {n}_{r} + 1 to {n}_{r} + {n}_{p}\end{matrix}\right.$ ${n}_{{e}_{j}} = \left\{\begin{matrix}{n}_{{r}_{j}} & j = 1 to {n}_{r} \\ {n}_{{p}_{j - {n}_{r}}} & j = {n}_{r} + 1 to {n}_{r} + {n}_{p}\end{matrix}\right.$

nₑ denotes the total number of reactants and products.

*r(C)* denotes the rank of matrix *C*. If:

*r(C)* = min(*m, nₑ*): there is only a unique zero solution, and the solution is meaningless;

*r(C)* = *min*(*m, nₑ*) - 1: there are infinitely many non-zero solutions, with a unique simplest integer-ratio solution;

*r(C)* < min(*m*, *nₑ*) - 1: there are infinitely many non-zero solutions, with infinitely many simplest integer-ratio solutions.

The above constitutes the chemical equation balancing algorithm.

The combination and decomposition of chemical reactions are crucial for describing all chemical reactions of radionuclides in a complex system. Consider an arbitrary set of chemical equations in a complex system as follows: ${n}_{0 , 1} {R}_{1} + ⋯ + {n}_{0 , {n}_{r}} {R}_{{n}_{r}} = - {n}_{0 , {n}_{r} + 1} {P}_{1} - ⋯ - {n}_{0 , {n}_{s}} {P}_{{n}_{p}}$ ${n}_{1 , 1} {R}_{1} + ⋯ + {n}_{1 , {n}_{r}} {R}_{{n}_{r}} = - {n}_{1 , {n}_{r} + 1} {P}_{1} - ⋯ - {n}_{1 , {n}_{s}} {P}_{{n}_{p}}$ ${n}_{k , 1} {R}_{1} + ⋯ + {n}_{k , {n}_{r}} {R}_{{n}_{r}} = - {n}_{k , {n}_{r} + 1} {P}_{1} - ⋯ - {n}_{k , {n}_{s}} {P}_{{n}_{p}}$

Where, *nᵢ,ᵣⱼ* and *n_{i,pj}* are the stoichiometric coefficients of reactants and products, respectively, in the chemical equation. The mass conservation of the above set of chemical equations may be described as: ${n}_{0 , j} = {\sum }_{i = 1}^{k} {c}_{i} {n}_{i , j} , j = 1 , … , {n}_{s}$ ${n}_{i , j} = \left\{\begin{matrix}{n}_{i , {r}_{j}} & j = 1 to {n}_{r} \\ {n}_{i , {p}_{j - {n}_{r}}} & j = {n}_{r} + 1 to {n}_{s}\end{matrix}\right.$

Where, *nₛ* = *nᵣ* + *nₚ* denotes the total number of components in the complex system. For the linear space $Ω = {ℚ}^{{n}_{s} - 1}$ of all chemical equations and the linear space of simple chemical equations *W* ⊆ *Ω,* if the first chemical equation in the above chemical equation set is selected, i.e., equation 0 is selected as any chemical equation in the entire multiphase system, and equations 1 to *k* are selected as all simple chemical equations, the matrix expression is: ${N}_{s}^{T} {C}_{s} = 0$ ${C}_{s} = \left(\begin{matrix}{c}_{0} \\ {c}_{1} \\ ⋯ \\ {c}_{k}\end{matrix}\right) , {c}_{0} = - 1$ ${N}_{s} = \left(\begin{matrix}{n}_{0 , 1} & {n}_{0 , 2} & ⋯ & {n}_{0 , {n}_{s}} \\ {n}_{1 , 1} & {n}_{1 , 2} & ⋯ & {n}_{1 , {n}_{s}} \\ ⋯ & ⋯ & ⋯ & ⋯ \\ {n}_{k , 1} & {n}_{k , 2} & ⋯ & {n}_{k , {n}_{s}}\end{matrix}\right)$

The solution of the above equation depends on the rank *r(Nₛ)* of the coefficient matrix *Nₛ* of the linear equation system, as shown below:
*r*(*Nₛ*) = min(k + 1, *nₛ*): there is only a unique zero solution, and the solution is meaningless;
*r*(*Nₛ*) = *min(k +* 1, *nₛ*) - 1: there are infinitely many non-zero solutions, with a unique simplest integer-ratio solution;
*r*(*Nₛ*) < min(*k* + 1, *nₛ*) - 1: there are infinitely many non-zero solutions, with infinitely many simplest integer-ratio solutions.

As mentioned above, equation 0 is regarded as any chemical equation in the entire complex system, and chemical equations 1 to *k* are selected as all basic chemical equations. Then, *W = Ω* indicates that chemical equation 0 can be obtained by combining chemical equations 1 to k, which means that the following equation has a non-zero solution. $\left(\begin{matrix}{n}_{0 , 1} \\ {n}_{0 , 2} \\ ⋯ \\ {n}_{0 , {n}_{s}}\end{matrix}\right) = {c}_{1} \left(\begin{matrix}{n}_{1 , 1} \\ {n}_{1 , 2} \\ ⋯ \\ {n}_{1 , {n}_{s}}\end{matrix}\right) + {c}_{2} \left(\begin{matrix}{n}_{2 , 1} \\ {n}_{2 , 2} \\ ⋯ \\ {n}_{2 , {n}_{s}}\end{matrix}\right) + ⋯ + {c}_{k} \left(\begin{matrix}{n}_{k , 1} \\ {n}_{k , 2} \\ ⋯ \\ {n}_{k , {n}_{s}}\end{matrix}\right)$ $α = {\sum }_{i = 1}^{k} {c}_{i} {β}_{i}$ $α = \left(\begin{matrix}{n}_{0 , 1} \\ {n}_{0 , 2} \\ ⋯ \\ {n}_{0 , {n}_{s}}\end{matrix}\right) , {β}_{i} = \left(\begin{matrix}{n}_{i , 1} \\ {n}_{i , 2} \\ ⋯ \\ {n}_{i , {n}_{s}}\end{matrix}\right) , i = 1 … , k$

Where, $α ∈ Ω = {ℚ}^{{n}_{s} - 1}$*.* Let *W* =< *β*₁,*β*₂, ... , *βₖ* >⊆ *Ω,* where W is within the linear space determined by *β*₁*, β*₂*,* ... , *βₖ* ; and let ${β}_{i} = \left(\begin{matrix}{n}_{i , 1} \\ {n}_{i , 2} \\ ⋯ \\ {n}_{i , {n}_{s}}\end{matrix}\right) ∈ {ℚ}^{{n}_{s}}$, *β*ᵢ represents the stoichiometric coefficient vector of chemical equation i. The following four conditions are generally satisfied:
${\sum }_{j = 1}^{{n}_{s}} {n}_{i , j} = 0$, represents the balance of the chemical equation;
*k* ≥ *nₛ* - 1, this means that the number of chemical equations is greater than the number of components in the system;
$∀ j ∈ \left[1, {n}_{s}\right] ∩ ℕ , \left(∃i∈\left[1, k\right]∩ℕ,{n}_{i , j}\neq 0\right)$, this means that each component is present in at least one chemical equation;
*β*ᵢ has at most four non-zero coordinates, which means that chemical equation i has at most three products or three reactants.

Therefore, *d*(*W*) = *d*(Ω) = *nₛ* - 1 and *W* ⊆ *Ω,* that is, *W = Ω.* If *k'* ≥ *nₛ* - 1, this indicates that the entire reaction system involves the chemical reaction having one to two reactants and products.

Then, according to the combination and decomposition of reactants, the simple chemical equation in the system is screened out, namely, the chemical equation in which the total number of reactants and products is less than or equal to four, and in most cases there are only one or two reactants. All other chemical equations are reconstructed from the simple chemical equation.

For the screened balanced simple chemical reaction equations: ${\sum }_{R} {ν}_{R} R = {\sum }_{P} {ν}_{P} P$

Where, R and P respectively denote the reactant and the product, and *ν_{R}* and *ν_{P}* respectively denote the corresponding stoichiometric coefficients. The molar Gibbs free energy change Δ*ᵣG_{m,T}* of the chemical reaction is: ${Δ}_{r} {G}_{m , T} = {\sum }_{P} {ν}_{P} {Δ}_{f} {G}_{m , T} \left(P\right) - {\sum }_{R} {ν}_{R} {Δ}_{f} {G}_{m , T} \left(R\right)$

Δ*_{f}G_{m,T}*(*R*) and Δ*_{f}G_{m,T}*(*P*) respectively denote the molar Gibbs free energies of formation of the reactant and the product, and Δ*ᵣG_{m,T}* denotes the molar Gibbs free energy change of the chemical reaction. By substituting the data in the g0table into the above equation, the Gibbs free energy change of the screened chemical reaction can be obtained, and it is determined whether Δ*ᵣG_{m,T}* is greater than 0; if it is less than 0, the chemical reaction can occur spontaneously.

In order to further improve the reliability of obtaining the key condition, in one embodiment of the present application, Step 100 may include: inputting a first input file and a input condition; and applying the first input file and the input condition to generate a second input file, and each record in the second input file may correspond to one of the above key conditions.

In order to determine the chemical reaction occurring under a plurality of key influencing parameters, thereby facilitating subsequent analysis of the effects of the key influencing parameters on the chemical states in the multiphase system, in one embodiment, a plurality of key conditions are provided, and the key influencing parameter of the multiphase system include at least one selected from a impurity element among the plurality of elements, the temperature, and the pressure. The values of the key influencing parameter corresponding to the respective key conditions are different. Correspondingly, as shown in Figure 5, after Step 100, the method further includes:
Step 400: predicting and obtaining the chemical state information under each key condition by using a Gibbs free energy minimization method.
Step 500: predicting and obtaining the chemical reaction that will occur in the multiphase system under each key condition based on the chemical state information under each key condition.

Specifically, each key condition includes temperature, pressure, a plurality of elements, and respective amounts thereof. The key influencing parameter corresponding to the respective key conditions may be the same, and the values of the corresponding key influencing parameter may be the same. For example, the key influencing parameters of the multiphase system include temperature and the carbon element C. In one key condition *a*, the key influencing parameters are temperature and the carbon element, the temperature value is 2.00E+02 °C, and the amount of the carbon element is 30.75 mol. In another key condition *b*, the key influencing parameters are temperature and the carbon element, the temperature value is 2.00E+02 °C, and the amount of the carbon element is 29.41 mol. The amounts of the carbon element in key condition *a* and key condition *b* are different. The values of the key influencing parameter corresponding to the respective key conditions are different, while the other values are the same.

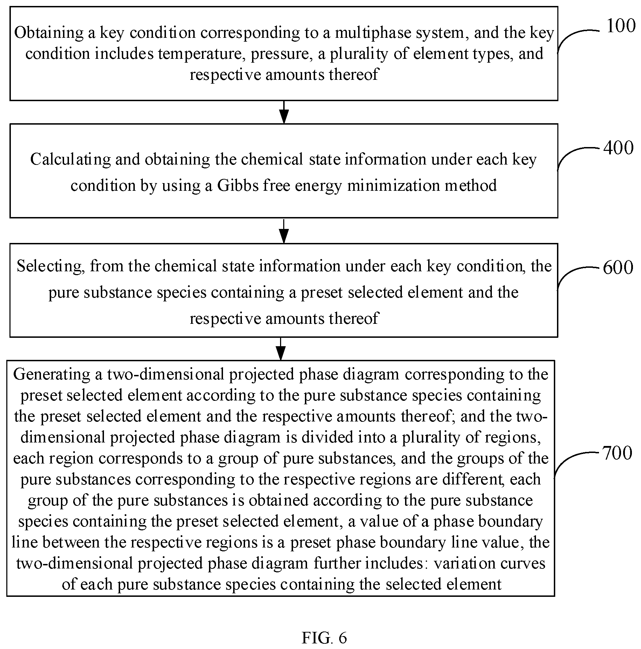

In order to improve the reliability of phase diagram generation and facilitate subsequent analysis, as shown in Figure 6, in one embodiment, after Step 400, the method further includes:
Step 600: selecting, from the chemical state information under each key condition, the pure substance species containing a preset selected element and the respective amounts thereof.
Step 700: generating a two-dimensional projected phase diagram corresponding to the preset selected element according to the pure substance species containing the preset selected element and the respective amounts thereof; and the two-dimensional projected phase diagram is divided into a plurality of regions, each region corresponds to a group of pure substances, and the groups of the pure substances corresponding to the respective regions are different, each group of the pure substances is obtained according to the pure substance species containing the preset selected element, a value of a phase boundary line between the respective regions is a preset phase boundary line value, the two-dimensional projected phase diagram further includes: variation curves of each pure substance species containing the selected element.

Specifically, the preset phase boundary line value can be set according to actual conditions, and the present application is not limited thereto. Exemplarily, the preset phase boundary line value is 0.01.

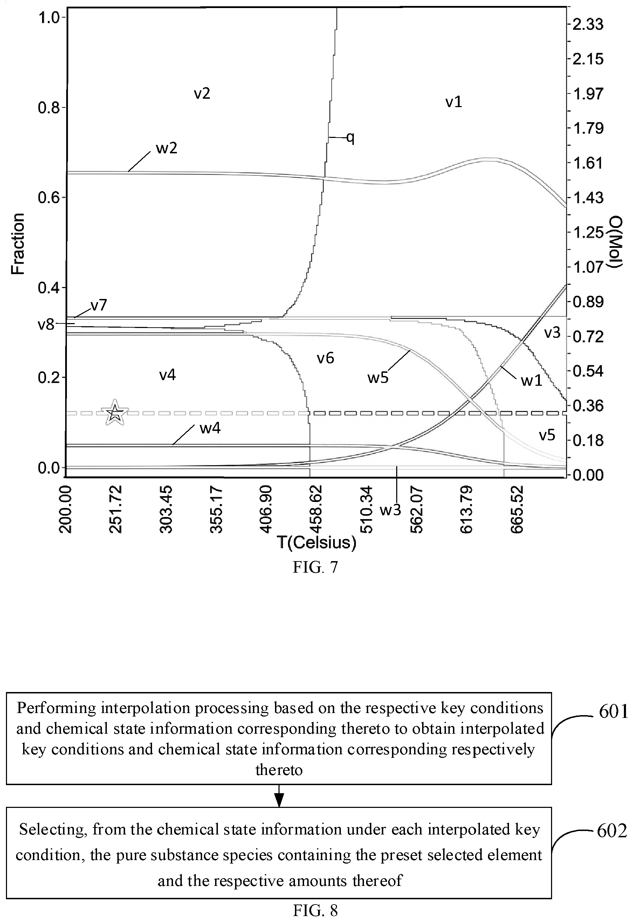

In one example, a two-dimensional projected phase diagram of iodine is shown in Figure 7. In Figure 7, w1 to w5 sequentially represent variation curves corresponding to the pure substance species I(g), HI(g), HIO(g), IRb(g), and CsI(g). The horizontal axis represents temperature, in degrees Celsius (°C). The vertical axis on the left side of the phase diagram corresponds to the variation curves and represents the relative amounts of the respective pure substances. v1 to v8 sequentially represent the following group of pure substances: I(g), HIO (g)+ I(g), HI(g)+ I(g), CsI(g)+ HI(g)+ IRb(g), CsI(g)+ HI(g)+ I(g), CsI(g)+ HI(g)+ I(g)+ IRb(g), Csl(g)+ HI(g)+ HIO (g)+ I(g), Csl(g)+ HI(g)+ HIO (g)+ I(g)+ IRb(g). The vertical axis on the right side of the phase diagram corresponds to the regions and represents the oxygen content, in moles (mol). In this example, the phase boundary line between region v1 and region v2 is q. The variation curves in the two-dimensional projected phase diagram may correspond to an upper-layer phase diagram, and the respective regions in the two-dimensional projected phase diagram may correspond to a lower-layer phase diagram. In the lower-layer phase diagram, a star symbol may be used to indicate a reference condition of two system variables. Since different operating conditions exist in the HTR-PM system, for one of the operating conditions, the temperature, pressure, and the amounts of impurity C, H, O, and N under that operating condition may be set. The function of setting the reference conditions is that, when the relationship between two system variables is considered, such as the temperature and O content in Figure 7, the remaining variables (for example, pressure and the amounts of C, H, and N) are taken as the reference conditions. In Figure 7, the position indicated by the star symbol represents the preset reference conditions of the system. The dashed line represents the reference condition of the system variable described by the Y-axis in the upper-layer phase diagram. For example, the dashed line indicates variations of the respective pure substances with temperature under the condition that the oxygen content is 0.35 mol.

In order to ensure the integrity of the chemical state information, as shown in Figure 8, in one embodiment, Step 600 includes:
Step 601: performing interpolation processing based on the respective key conditions and chemical state information corresponding thereto to obtain interpolated key conditions and chemical state information corresponding respectively thereto.
Step 602: selecting, from the chemical state information under each interpolated key condition, the pure substance species containing the preset selected element and the respective amounts thereof.

Specifically, based on an interpolation algorithm, the respective key conditions and the chemical state information corresponding thereto, interpolated key conditions and the chemical state information corresponding respectively thereto may be obtained.

The interpolation algorithm may be as follows:
The N-dimensional matrix-table interpolation function *F(x₁,...,x_{N})* is constructed based on tabulated function values and is used to approximately calculate *f(x₁,...,x_{N}),* which is expressed as: $\left[f\left({X}_{{i}_{1}},{X}_{{i}_{2}},…,{X}_{{i}_{N}}\right):{i}_{1}=1,…,{n}_{1},…,{i}_{N}=1,…,{n}_{N}\right]$

The gradient of the interpolation function *F(x₁,...,x_{N}),* which gives an approximate value of the function *f(x₁,...,x_{N}),* is: $∇ F \left({x}_{1},…,{x}_{N}\right) = {\left({F}^{{x}_{1}},…,{F}^{{x}_{N}}\right)}^{T} = {\left(\frac{\partial F}{\partial {x}_{1}},…,\frac{\partial F}{\partial {x}_{N}}\right)}^{T}$

First, an appropriate table interval is determined: (*X*_{*I*1}*,X*_{*I*1+1})*,...,*(X*_{IN}, X*_{*IN+*1})*,* where *X*_{*I*1} ≤ *x*₁ ≤ *X*_{*I*1*+*1}*, ..., X_{IN}* ≤ *x_{N}* ≤ *X*_{*IN+*1}*,* and the interval Ω_{F} = {(*x*₁*, ..., X_{N}*): *X*_{*I*1} ≤ *x*₁ ≤ *X*_{*I*1+1}, *.., X_{IN}* ≤ *x_{N}* ≤ *X*_{*IN+*1}}. Each interval is then scaled to (0, 1), thereby transforming the problem into one of finding a matrix-table interpolation function *G(y₁,...,y_{N})* for *g(y₁,...,y_{N})* in an N-dimensional unit space, where the interval *Ω_{G}* = {(*y*₁, ..., *y_{N}*): 0 ≤ *y*₁ ≤ 1,..,0 ≤ *y_{N}* ≤ 1}.

The transformed N-dimensional matrix table interpolation function *G(y₁,...,y_{N})* is determined by the following equation: $\left[g\left({j}_{1},…,{j}_{N}\right):{j}_{1}=0 or 1,…,{j}_{N}=0 or 1\right]$ ${y}_{i} = \frac{{x}_{i} - {X}_{{I}_{i}}}{{X}_{{I}_{i} + 1} - {X}_{{I}_{i}}} and g \left({j}_{1},…,{j}_{N}\right) = f \left({X}_{{I}_{1} + {j}_{1}},…,{X}_{{I}_{N} + {j}_{N}}\right)$

Where the piecewise linear interpolation *F_{L}* is computed as a simplex with complexity N!. ${S}_{p \left(1\right) , … , p \left(N\right)} = \left[\left({y}_{1},…,{y}_{N}\right):0\leq {y}_{p \left(1\right)}\leq ⋯\leq {y}_{p \left(N\right)}\leq 1\right]$

Where (p(1), p(2), ..., p(N)) is a permutation of integers in the range from 1 to N. The simplex is determined by the ordering coefficients *y₁,* ..., *y_{N}.* The form of *F_{L}* is: ${F}_{L} = {l}_{0} + {l}_{1} {y}_{1} + ⋯ + {l}_{N} {y}_{N}$
where *lᵢ* is defined as a coefficient matching *f* at the vertices *s₀* and *s_{N}* of the simplex, and the vertex *sᵢ* has a value of 1 at position *p(j)* for j>i, and 0 at all other positions.

For each z=(*x₁,...,x_{N}*)^{T} within the range of Ω_{F} , the error between the interpolation function and the original function is: $\left|\left(f-{F}_{L}\right)\left(z\right)\right| \leq \frac{N}{8} {h}^{2} {sup}_{{θ}^{T} θ = 1 , x} \left|{D}_{θ}^{2}f\left(x\right)\right|$

Where *D_{θ}f*(*x*) is the directional derivative of *f(x)* in the *θ* direction.

In order to further illustrate the present solution, the present application provides a chemical reaction detection method for a multiphase system, which is specifically described as follows:
S1: performing parameter configuration and data preprocessing.

First, before the calculation, key parameters are set, including module control parameters (the calculation process is divided into two modules, and the phase-diagram plotting module and the reaction analysis module are respectively enabled according to calculation requirements). Second, a user input file with a specific format (Template.equi) required for FactSage operation and calculation parameters (input.txt) are input. Finally, corresponding file storage paths are established, including paths for input files, output files, and the like. The function implemented by the user input file can correspond to that of the above first input file, and the function implemented by the calculation parameters can correspond to that of the above input conditions.

The most critical input conditions in the user input file (i.e.,Template.equi) are the types and amounts of elements considered in the multiphase system. In one example, the input conditions in the user input file include:
6.18E-08Kr +3.82E-07Xe +2.58E-10I +6.40E-13Sr +5.96E-10Cs +6.73E-12Ag +1.75E-11Co +3.88E-13Fe +3.73E-15Cr +1.81E-15Mn +3.33E-13Ni +7.93E-12Rb +750000He +<Variable_C>C +<Variable_H>H +<Variable_O>O +<Variable_N>N
where <Variable_X>X represents an impurity element X in the multiphase system and the amount thereof, and X may be C, H, O, or N, with the amount thereof being given in the calculation parameters.

The calculation parameters Y include temperature, pressure, and the respective impurity elements, and the key influencing parameters of the multiphase system may include at least one of the temperature, the pressure, and the respective impurity elements. In one example, the corresponding relationship among the calculation parameter Y, the lower limit *a* of a variation interval, the upper limit b of the variation interval, and the number c of uniformly sampled values within the variation interval is shown in Table 1. In this example, the number of uniformly sampled values within the variation interval of temperature is 26, which may indicate that 26 temperature values are taken between 200 °C and 1000 °C with a fixed interval; the number of uniformly sampled values within the variation interval of pressure is 1, which indicates that the pressure remains constant at 0.005 GPa; the number of uniformly sampled values within the variation interval of the impurity element C is 24, which indicates that 24 amount values are taken between 0 mol and 30.75 mol with a fixed interval; and the numbers of uniformly sampled values within the variation intervals of the impurity elements H, O, and N are all 1, which indicates that the amounts thereof remain constant, the amounts of the impurity elements H, O, and N being 4.99 mol, 6.11 mol, and 0.2 mol, respectively, and the key influencing parameters of the multiphase system are temperature T and impurity element C.

**Table 1**

| Calculation parameters | Lower limit of the variation interval | Upper limit of the variation interval | Number of uniform sampled points within the variation interval |
|---|---|---|---|
| Temperature T | 200°C | 1000°C | 26 |
| Pressure P | 0.005GPa | 0.005GPa | 1 |
| Impurity element C | 0 mol | 30.75 mol | 24 |
| Impurity element H | 0 mol | 4.99 mol | 1 |
| Impurity element O | 0 mol | 6.11 mol | 1 |
| Impurity element N | 0 mol | 0.2 mol | 1 |

S2: invoking the FactSage software for execution.

A FactSage input file (*.equi) is generated according to the user input file Template.equi and the calculation parameters input.txt. The function implemented by the FactSage input file may correspond to that of the above second input file.

In the above example, the key influencing parameters of the multiphase system are temperature T and the impurity element C, and temperature T takes 26 values and the impurity element C takes 24 values. Therefore, 24 FactSage input files can be generated, and the amounts of the impurity element C corresponding to the respective FactSage input files are different. The amount of the impurity element C in the same FactSage input file is the same, and the same FactSage input file includes 26 temperature values, that is, the same FactSage input file contains 26 records, and the temperature values of the respective records are different while the other data are the same. One FactSage input file includes: Temperature: (2.00E+02)°C, (2.33E+02)°C, (2.67E+02)°C...; Pressure: 5.00E-03GPa; 6.18E-08Kr +3.82E-07Xe +2.58E-10I +6.40E-13Sr +5.96E-10Cs +6.73E-12Ag +1.75E-11Co +3.88E-13Fe +3.73E-15Cr +1.81E-15Mn +3.33E-13Ni +7.93E-12Rb +750000He +1.3C +4.99H +6.110 +0.2N, unit of amount (mol).

The FactSage input file is applied to invoke the macros module of FactSage to perform the Equilib equilibrium calculation, thereby obtaining a FactSage calculation result file (.tab). The FactSage input files correspond one-to-one with the .tab files. Taking one .tab file as an example, considering that the types of elements in the multiphase system are different, the pure substance species present in the multiphase system may also differ. After calculation, a total of 406 pure substance species (including compounds and elemental substances) exists in the present multiphase system, all of which are output to the .tab file, as shown in Table 2. Here, only a portion of one calculation result, i.e., one .tab file, is listed for illustration. Each row in Table 2 may correspond to the temperature, pressure, and amounts of the respective pure substances under one of the above key conditions.

**Table 2**

| T(°C) | P(GPa) | Mol-H(g) | Mol-H2(g) | Mol-He(g) | Mol-C(g) | Mol-C2(g) | ...... |
|---|---|---|---|---|---|---|---|
| 2.00E+02 | 5.00E-03 | 2.88E-20 | 4.52E-01 | 7.50E+05 | 1.17E-67 | 0.00E+00 | |
| 2.33E+02 | 5.00E-03 | 1.50E-18 | 7.97E-01 | 7.50E+05 | 1.94E-62 | 0.00E+00 | |
| 2.67E+02 | 5.00E-03 | 4.57E-17 | 1.19E+00 | 7.50E+05 | 7.31E-58 | 4.73E-67 | |
| 3.00E+02 | 5.00E-03 | 9.00E-16 | 1.57E+00 | 7.50E+05 | 8.11E-54 | 2.36E-62 | |
| 3.33E+02 | 5.00E-03 | 1.24E-14 | 1.86E+00 | 7.50E+05 | 3.24E-50 | 3.58E-58 | |
| 3.67E+02 | 5.00E-03 | 1.27E-13 | 2.08E+00 | 7.50E+05 | 5.45E-47 | 2.00E-54 | |
| 4.00E+02 | 5.00E-03 | 1.02E-12 | 2.23E+00 | 7.50E+05 | 4.41E-44 | 4.75E-51 | |
| 4.33E+02 | 5.00E-03 | 6.71E-12 | 2.34E+00 | 7.50E+05 | 1.90E-41 | 5.42E-48 | |
| 4.67E+02 | 5.00E-03 | 3.70E-11 | 2.41E+00 | 7.50E+05 | 4.72E-39 | 3.28E-45 | |
| 5.00E+02 | 5.00E-03 | 1.75E-10 | 2.45E+00 | 7.50E+05 | 7.31E-37 | 1.14E-42 | |
| 5.33E+02 | 5.00E-03 | 7.30E-10 | 2.48E+00 | 7.50E+05 | 7.46E-35 | 2.45E-40 | |
| 5.67E+02 | 5.00E-03 | 2.71E-09 | 2.49E+00 | 7.50E+05 | 5.28E-33 | 3.43E-38 | |
| 6.00E+02 | 5.00E-03 | 9.10E-09 | 2.49E+00 | 7.50E+05 | 2.70E-31 | 3.29E-36 | |
| 6.33E+02 | 5.00E-03 | 2.80E-08 | 2.49E+00 | 7.50E+05 | 1.03E-29 | 2.25E-34 | |
| 6.67E+02 | 5.00E-03 | 7.94E-08 | 2.49E+00 | 7.50E+05 | 3.05E-28 | 1.14E-32 | |
| 7.00E+02 | 5.00E-03 | 2.10E-07 | 2.49E+00 | 7.50E+05 | 7.15E-27 | 4.43E-31 | |
| 7.33E+02 | 5.00E-03 | 5.22E-07 | 2.49E+00 | 7.50E+05 | 1.36E-25 | 1.35E-29 | |
| 7.67E+02 | 5.00E-03 | 1.22E-06 | 2.49E+00 | 7.50E+05 | 2.14E-24 | 3.28E-28 | |
| 8.00E+02 | 5.00E-03 | 2.72E-06 | 2.49E+00 | 7.50E+05 | 2.84E-23 | 6.56E-27 | |
| 8.33E+02 | 5.00E-03 | 5.77E-06 | 2.49E+00 | 7.50E+05 | 3.22E-22 | 1.09E-25 | |
| 8.67E+02 | 5.00E-03 | 1.17E-05 | 2.49E+00 | 7.50E+05 | 3.17E-21 | 1.55E-24 | |
| 9.00E+02 | 5.00E-03 | 2.29E-05 | 2.49E+00 | 7.50E+05 | 2.74E-20 | 1.88E-23 | |
| 9.33E+02 | 5.00E-03 | 4.31E-05 | 2.49E+00 | 7.50E+05 | 2.10E-19 | 1.98E-22 | |
| 9.67E+02 | 5.00E-03 | 7.85E-05 | 2.49E+00 | 7.50E+05 | 1.44E-18 | 1.85E-21 | |
| ...... | | | | | | | |
| 1.00E+03 | 5.00E-03 | 1.38E-04 | 2.49E+00 | 7.50E+05 | 8.96E-18 | 1.53E-20 | |

S3: extracting key data from the calculation results to obtain reactants related to reaction conditions, reactant data, amount data (datas) of chemical states of all elements in the multiphase system under different conditions, and Gibbs free energy data (g0table). For the dataset (datas), the variables are divided into reactant-amount and condition data (Xtable), and amount data of all pure substance species in the multiphase system under the corresponding conditions (Ytable). In this step, it is particularly important to ensure the integrity of the data obtained from FactSage calculations, because due to the large computational workload, FactSage may occasionally lose a small amount of data, in which case interpolation is employed to manually supplement the missing data.

In one example, the reactants can be extracted according to Table 1, as shown in Table 3, where the unit of amount is mol.

**Table 3**

| C | H | O | N |
|---|---|---|---|
| 30.75 | 4.99 | 6.11 | 0.20 |
| 29.41 | 4.99 | 6.11 | 0.20 |
| 28.08 | 4.99 | 6.11 | 0.20 |
| 26.74 | 4.99 | 6.11 | 0.20 |
| 25.40 | 4.99 | 6.11 | 0.20 |
| 24.07 | 4.99 | 6.11 | 0.20 |
| 22.73 | 4.99 | 6.11 | 0.20 |
| 21.39 | 4.99 | 6.11 | 0.20 |
| 20.05 | 4.99 | 6.11 | 0.20 |
| 18.72 | 4.99 | 6.11 | 0.20 |
| 17.38 | 4.99 | 6.11 | 0.20 |
| 16.04 | 4.99 | 6.11 | 0.20 |
| 14.71 | 4.99 | 6.11 | 0.20 |
| 13.37 | 4.99 | 6.11 | 0.20 |
| 12.03 | 4.99 | 6.11 | 0.20 |
| 10.70 | 4.99 | 6.11 | 0.20 |
| 9.36 | 4.99 | 6.11 | 0.20 |
| 8.02 | 4.99 | 6.11 | 0.20 |
| 6.68 | 4.99 | 6.11 | 0.20 |
| 5.35 | 4.99 | 6.11 | 0.20 |
| 4.01 | 4.99 | 6.11 | 0.20 |
| 2.67 | 4.99 | 6.11 | 0.20 |
| 1.34 | 4.99 | 6.11 | 0.20 |
| 0.00 | 4.99 | 6.11 | 0.20 |

In this example, Gibbs free energy values of the 406 pure substance species at different temperatures are extracted to obtain the Gibbs free energy data (g0table), as exemplified in Table 4. In Table 4, G is an abbreviation for Gibbs free energy, J denotes the unit joule, g denotes gas, and H, H₂, He, C, and C2 denote substances.

**Table 4**

| T(°C) | P(GPa) | G(J)-H(g) | G(J)-H2(g) | G(J)-He(g) | G(J)-C(g) | G(J)-C2(g) | ...... |
|---|---|---|---|---|---|---|---|
| 200.00 | 0.005 | 178205.8 | -47704.3 | -45203.2 | 656359 | 750718.9 | |
| 233.33 | 0.005 | 175122.2 | -51457.5 | -48667.9 | 651828.8 | 744533.7 | |
| 266.67 | 0.005 | 171993 | -55274.9 | -52178.2 | 647252.8 | 738262 | |
| 300.00 | 0.005 | 168821 | -59152.6 | -55731.4 | 642634 | 731910.4 | |
| 333.33 | 0.005 | 165608.7 | -63087.3 | -59324.8 | 637974.9 | 725484.7 | |
| 366.67 | 0.005 | 162358.3 | -67075.7 | -62956.3 | 633277.6 | 718989.7 | |
| 400.00 | 0.005 | 159071.8 | -71115.1 | -66624 | 628544.2 | 712429.6 | |
| 433.33 | 0.005 | 155750.9 | -75203.2 | -70326 | 623776.5 | 705808 | |
| 466.67 | 0.005 | 152397.4 | -79337.6 | -74060.7 | 618976 | 699128.1 | |
| 500.00 | 0.005 | 149012.6 | -83516.4 | -77826.6 | 614144.4 | 692392.9 | |
| 533.33 | 0.005 | 145598 | -87737.6 | -81622.4 | 609282.8 | 685604.8 | |
| 566.67 | 0.005 | 142154.7 | -91999.8 | -85446.8 | 604392.6 | 678766.2 | |
| 600.00 | 0.005 | 138683.8 | -96301.3 | -89298.8 | 599475 | 671879.2 | |
| 633.33 | 0.005 | 135186.6 | -100641 | -93177.2 | 594530.9 | 664945.6 | |
| 666.67 | 0.005 | 131663.8 | -105017 | -97081.1 | 589561.3 | 657967.2 | |
| 700.00 | 0.005 | 128116.5 | -109428 | -101010 | 584567.1 | 650945.7 | |
| 733.33 | 0.005 | 124545.4 | -113874 | -104962 | 579549.3 | 643882.4 | |
| 766.67 | 0.005 | 120951.4 | -118353 | -108937 | 574508.5 | 636778.7 | |
| 800.00 | 0.005 | 117335.1 | -122865 | -112934 | 569445.5 | 629635.8 | |
| 833.33 | 0.005 | 113697.4 | -127408 | -116953 | 564360.9 | 622455.1 | |
| 866.67 | 0.005 | 110038.7 | -131982 | -120993 | 559255.6 | 615237.5 | |
| 900.00 | 0.005 | 106359.8 | -136586 | -125053 | 554129.9 | 607984 | |
| 933.33 | 0.005 | 102661.2 | -141219 | -129133 | 548984.6 | 600695.7 | |
| 966.67 | 0.005 | 98943.49 | -145881 | -133232 | 543820.1 | 593373.5 | |
| 983.34 | 0.005 | 97075.31 | -148225 | -135290 | 541228.6 | 589695.8 | |
| 1000.00 | 0.005 | 95207.12 | -150570 | -137349 | 538637 | 586018.1 | |

Xtable summarizes the amounts of the respective calculation parameters under all key conditions. Xtable is a 624×6 data table, as shown in Table 5, of which only a portion is listed for illustration.

**Table 5**

| Number | T(°C) | P(GPa) | C(Mol) | H(Mol) | O(Mol) | N(Mol) |
|---|---|---|---|---|---|---|
| 1 | 200.00 | 0.005 | 30.75 | 4.99 | 6.11 | 0.20 |
| 2 | 233.33 | 0.005 | 30.75 | 4.99 | 6.11 | 0.20 |
| 3 | 266.67 | 0.005 | 30.75 | 4.99 | 6.11 | 0.20 |
| 4 | 300.00 | 0.005 | 30.75 | 4.99 | 6.11 | 0.20 |
| 5 | 333.33 | 0.005 | 30.75 | 4.99 | 6.11 | 0.20 |
| 6 | 366.67 | 0.005 | 30.75 | 4.99 | 6.11 | 0.20 |
| 7 | 400.00 | 0.005 | 30.75 | 4.99 | 6.11 | 0.20 |
| 8 | 433.33 | 0.005 | 30.75 | 4.99 | 6.11 | 0.20 |
| 9 | 466.67 | 0.005 | 30.75 | 4.99 | 6.11 | 0.20 |
| 10 | 500.00 | 0.005 | 30.75 | 4.99 | 6.11 | 0.20 |
| 11 | 533.33 | 0.005 | 30.75 | 4.99 | 6.11 | 0.20 |
| 12 | 566.67 | 0.005 | 30.75 | 4.99 | 6.11 | 0.20 |
| 13 | 600.00 | 0.005 | 30.75 | 4.99 | 6.11 | 0.20 |
| 14 | 633.33 | 0.005 | 30.75 | 4.99 | 6.11 | 0.20 |
| 15 | 666.67 | 0.005 | 30.75 | 4.99 | 6.11 | 0.20 |
| 16 | 700.00 | 0.005 | 30.75 | 4.99 | 6.11 | 0.20 |
| 17 | 733.33 | 0.005 | 30.75 | 4.99 | 6.11 | 0.20 |
| 18 | 766.67 | 0.005 | 30.75 | 4.99 | 6.11 | 0.20 |
| 19 | 800.00 | 0.005 | 30.75 | 4.99 | 6.11 | 0.20 |
| 20 | 833.33 | 0.005 | 30.75 | 4.99 | 6.11 | 0.20 |
| 21 | 866.67 | 0.005 | 30.75 | 4.99 | 6.11 | 0.20 |
| 22 | 900.00 | 0.005 | 30.75 | 4.99 | 6.11 | 0.20 |
| 23 | 933.33 | 0.005 | 30.75 | 4.99 | 6.11 | 0.20 |
| 24 | 966.67 | 0.005 | 30.75 | 4.99 | 6.11 | 0.20 |
| 25 | 983.34 | 0.005 | 30.75 | 4.99 | 6.11 | 0.20 |
| 26 | 1000.00 | 0.005 | 30.75 | 4.99 | 6.11 | 0.20 |
| 27 | 200.00 | 0.005 | 29.41 | 4.99 | 6.11 | 0.20 |
| ...... | | | | | | |
| 624 | 1000.00 | 0.005 | 0.00 | 4.99 | 6.11 | 0.20 |

Ytable summarizes the types and amounts of pure substance species in the multiphase system under all key conditions. Ytable is a 624×406 data table and may be constructed from the respective .tab files.

S4: plotting a phase diagram.

Since a large number of pure substance species are considered in a complex multiphase system, when plotting the phase diagram, it is necessary to provide a threshold for the relative percentage of the pure substances, and to determine, by screening according to the amounts, whether the corresponding pure substances exist in the complex multiphase system. If the relative percentage of a pure substance is greater than the preset threshold, the pure substance is considered to exist.

The plotting parameters are set as condition: [250, 0.005, 30.75, 4.99, 6.11, 0.20], which respectively represent temperature, pressure, and the amounts of C, H, O, and N.

The X-axis and the Y-axis are selected, where the X-axis is temperature T and the Y-axis is the amount of O. After the X-axis and the Y-axis are selected, the amounts of the calculation parameters pressure, C, H, and N are [0.005, 30.75, 4.99, 0.20] in sequence from the above plotting parameters.

The selected element is iodine (I), i.e., the O-T phase diagram of element I is displayed in the phase diagram.

The interpolation density is [300, 300].

Based on the interpolation algorithm mentioned above, since the selected interpolation density is [300, 300] and the X-axis and the Y-axis have been selected, after interpolation processing, Xtable and Ytable become cons_Xtable and cons_Ytable, respectively. The cons_Xtable is a 90000×2 data table, and cons_Ytable is a 90000×406 data table. After interpolation, the amount of data increases, thereby facilitating phase diagram plotting.

The two-dimensional projected phase diagram has two layers. The lower layer is similar to a conventional phase diagram and provides the chemical state composition of elements in the multiphase system, where two variables (the X-axis and the Y-axis) vary while the other variables in the phase diagram remain unchanged. Different regions represent different chemical state compositions. The boundary line between two chemical state compositions means that the relative percentage of one component in an adjacent region is equal to the preset threshold, i.e., 0.01, which is different from a phase boundary line in a conventional phase diagram. When the multiphase system variable represented by the Y-axis in the lower layer phase diagram is kept constant, the upper layer phase diagram describes the key chemical states of elements in the multiphase system as functions of the multiphase system variable represented by the X-axis, i.e., the variation trends of the relative amounts of the respective chemical states with respect to the X-axis. In the color plot, different chemical states in the upper layer phase diagram are represented by lines of different colors, and different chemical state compositions in the lower layer phase diagram are represented by color blocks. In addition, in the lower layer phase diagram, a star symbol is used to indicate the reference conditions of two variables of the multiphase system, and a dashed line is used to indicate the reference condition of the multiphase system variable represented by the Y-axis in the upper layer phase diagram.

S5: analyzing the chemical reaction.

According to the preset selected element, related pure substance species and chemical reactions are searched, possible basic chemical equations are returned, and, in combination with the above obtained g0table, potential basic chemical reactions in the chemical system are obtained through calculation of G0.

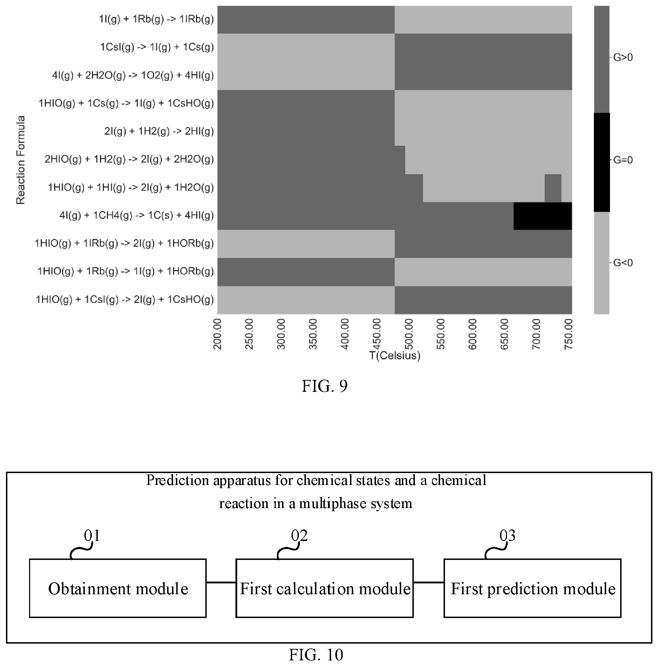

Specifically, after the chemical states are determined, the simple chemical equation is searched, which is defined as a chemical equation having one or two reactants and one or two products. Since there are an infinite number of chemical equations under the key conditions of the multiphase system, the simple chemical equation is listed, and other chemical equations in the multiphase system are constructed based on linear combinations of the simple chemical equation. By using the Gibbs free energy of the chemical equations, the simple chemical equation capable of occurring spontaneously is screened out from all basic chemical equations in the complex system, as shown in Figure 9. The vertical axis represents chemical reactions that the element I may undergo in the multiphase system as obtained by calculation, the horizontal axis represents temperature, and the color blocks represent variations in the Gibbs free energy of the chemical reactions.

The criteria for chemical reactions that may occur in a complex multiphase system are as follows:
(a) variation in the amount of substances within a certain range; (b) variation in the chemical states of reactants; and (c) occurrence of chemical reactions in the direction of ΔG<0.

Balancing the chemical equation is a method for determining a chemical equation. A balanced chemical equation must satisfy the rule of conservation of mass and accurately represent the mass ratios between reactants and products, thereby providing precise relationships for chemical calculations.

From the above description, it can be seen that the present embodiment can provide a method for performing batch calculations on a multiphase system based on the thermodynamic calculation software FactSage and for studying and analyzing the chemical states and reactions obtained from the calculations. The method can be used to analyze the chemical states of the multiphase system and to summarize and analyze chemical reactions that may occur in the system. By invoking the equilibrium calculation module of FactSage, thermodynamic equilibrium calculations can be completed in batch. A two-dimensional projected phase diagram of the system can be plotted, and the error of the phase diagram can be discussed while keeping other parameters fixed. Complex reactions and simple reactions that may occur in the chemical system can be determined, and the effects of changes in key influencing parameters on the chemical states of elements and chemical reactions can be discussed. The method can analyze the chemical states of complex multiphase systems and can further analyze and predict chemical reactions that may occur in the system. Compared with conventional two-dimensional phase diagrams that can only provide the composition of chemical phases, the present method not only provides information on chemical state composition in the phase diagram, but also simultaneously provides variation trends of the relative amounts of the respective chemical states with respect to the X-axis, thereby making the phase diagram information more comprehensive and facilitating subsequent analysis. In addition, more importantly, the present method can further analyze chemical reactions that may occur in the system. For phase diagram plotting, conventional approaches use a zero-phase line as the boundary, which on the one hand makes it difficult to describe multielement systems, and on the other hand provides limited information and lacks amount related information. In contrast, by setting a threshold, the present method can define the phase boundary as a 0.01 line or other user-defined phase boundary line, thereby enabling the phase diagram to clearly describe the chemical state composition in a multiphase system. As a result, the amount of information conveyed is significantly increased compared with conventional phase diagrams. In addition, by superimposing the upper layer phase diagram, the present method can clearly display variation trends of the amounts of the respective chemical states of elements. For chemical equation searching, the present method is based on a pure substance database determined from chemical states. First, all pure substance species related to the element of interest with relatively high amounts, together with the corresponding thermodynamic data, are screened. Then, according to the chemical balancing algorithm described above, possible chemical reactions in the multiphase system are enumerated. Subsequently, by calculating Gibbs free energy changes of the chemical reactions, reactions capable of occurring spontaneously are screened out. This method can be used to determine chemical states and possible chemical reactions in multicomponent and multiphase systems with different levels of elemental amounts, and can reflect underlying physical principles and describe detailed information of the processes.

From a software perspective, in order to improve the accuracy and efficiency of predicting the chemical reaction in a multiphase system on the basis of predicting the chemical states of elements in the multiphase system, the present application provides an embodiment of a prediction apparatus for chemical states and a chemical reaction in the multiphase system. Referring to Figure 10, the prediction apparatus for the chemical states and a chemical reaction in the multiphase system specifically includes the following:

Obtainment module 01 configured to obtain a key condition corresponding to the multiphase system, and the key condition includes temperature, pressure, a plurality of element types, and respective amounts thereof;

First calculation module 02 configured to predict and obtain chemical state information under the key condition by using a Gibbs free energy minimization method, and the chemical state information includes a plurality of pure substance species and respective amounts thereof when the multiphase system reaches thermodynamic equilibrium;

First prediction module 03 configured to predict and obtain a chemical reaction that will occur in the multiphase system under the key condition based on the chemical state information.

In one embodiment, a plurality of key conditions are provided, a key influencing parameter of the multiphase system include at least one selected from a impurity element type among the plurality of element types, temperature, and pressure, values of the key influencing parameter corresponding to the respective key conditions are different;

Correspondingly, the prediction apparatus for the chemical states and the chemical reaction in the multiphase system further includes:
Second calculation module configured to predict and obtain the chemical state information under each key condition by using a Gibbs free energy minimization method;
Second prediction module configured to predict and obtain the chemical reaction that will occur in the multiphase system under each key condition based on the chemical state information under each key condition.

In one embodiment, the prediction apparatus for the chemical states and the chemical reaction in the multiphase system further includes:
Extraction module configured to select, from the chemical-state information under each key condition, pure substance species containing a preset selected element and respective amounts thereof;
Phase diagram plotting module configured to generate a two-dimensional projected phase diagram corresponding to the preset selected element according to the pure substance species containing the preset selected element and the respective amounts thereof;
And the two-dimensional projected phase diagram is divided into a plurality of regions, each region corresponds to a group of pure substances, and the groups of the pure substances corresponding to the respective regions are different, each group of the pure substances is obtained according to the pure substance species containing the preset selected element, a value of a phase boundary line between the respective regions is a preset phase boundary line value, the two-dimensional projected phase diagram further includes respective variation curves of the pure substance species containing the selected element.

In one embodiment, the extraction module includes:
Interpolation processing unit configured to perform interpolation processing based on the respective key conditions and chemical state information corresponding thereto to obtain the interpolated key conditions and chemical state information corresponding respectively thereto;
Extraction unit configured to select, from the chemical state information under each interpolated key condition, the pure substance species containing the preset selected element and the respective amounts thereof.

The embodiments of the prediction apparatus for the chemical states and the chemical reaction in the multiphase system provided in this specification can specifically be used to execute the processing flow of the above-described prediction method for the chemical states and the chemical reaction in the multiphase system. The functions are not reiterated here and can be referred to in the detailed description of the embodiments of the prediction method for the chemical states and the chemical reaction in the multiphase system mentioned above.

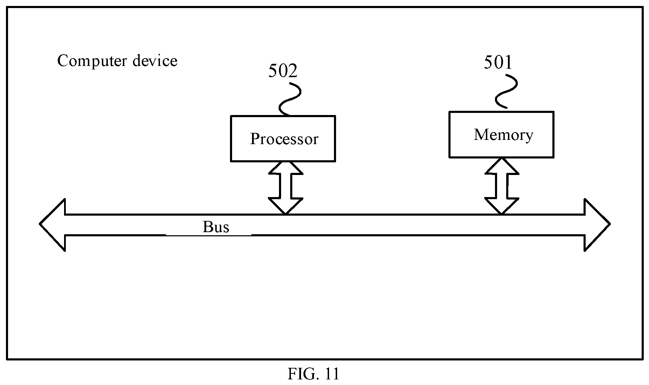

Figure 11 is a schematic diagram of the physical structure of the computer device provided in the embodiment of the present disclosure, as shown in Figure 11. The electronic device includes: a memory 501, a processor 502, and a computer program stored in memory 501 that can be executed on processor 502. When the processor 502 executes the computer program, the following method is implemented:
Step 100: obtaining a key condition corresponding to a multiphase system, and the key condition includes temperature, pressure, a plurality of element types, and respective amounts thereof.
Step 200: predicting and obtaining chemical state information under the key condition by using a Gibbs free energy minimization method, and the chemical state information includes a plurality of pure substance species in the multiphase system and the respective amounts thereof when the multiphase system reaches thermodynamic equilibrium.
Step 300: predicting and obtaining a chemical reaction that will occur in the multiphase system under the key condition based on the chemical state information.

This embodiment provides a computer-readable storage medium, and the computer-readable storage medium stores a computer program that, when executed by a processor, implements the following method:
Step 100: obtaining a key condition corresponding to a multiphase system, and the key condition includes temperature, pressure, a plurality of element types, and respective amounts thereof.
Step 200: predicting and obtaining chemical state information under the key condition by using a Gibbs free energy minimization method, and the chemical state information includes a plurality of pure substance species in the multiphase system and the respective amounts thereof when the multiphase system reaches thermodynamic equilibrium.
Step 300: predicting and obtaining a chemical reaction that will occur in the multiphase system under the key condition based on the chemical state information.

Those skilled in the art will appreciate that the embodiments of the present disclosure can be provided as a method, system, or computer program product. Therefore, the disclosure can take the form of an entirely hardware-based embodiment, an entirely software-based embodiment, or an embodiment combining software and hardware. Furthermore, the disclosure can be implemented in the form of a computer program product executed on one or more computer-readable storage mediums, which may include, but are not limited to, disk storage, CD-ROMs, optical storage, etc., containing computer-readable program code.

The present disclosure is described with reference to the flowcharts and/or block diagrams of the methods, devices (systems), and computer program products according to the embodiments of the present disclosure. It should be understood that each process and/or block in the flowchart and/or block diagram, as well as the combination of processes and/or blocks in the flowchart and/or block diagram, can be implemented by computer program instructions. These computer program instructions may be provided to a general-purpose computer, a special purpose computer, an embedded processor, or other programmable data processing devices to produce a machine, such that the instructions executed by the processor of the computer or other programmable data processing device result in a device that performs the functions specified in one or more processes in the flowchart or one or more blocks in the block diagram.

These computer program instructions may also be stored in a computer readable storage medium that can guide a computer or other programmable data processing device to operate in a specific manner, such that the instructions stored in the computer readable storage medium result in a manufactured product that includes an instruction device. This instruction device performs the functions specified in one or more processes in the flowchart or one or more blocks in the block diagram.

These computer program instructions may also be loaded onto a computer or other programmable data processing device, so that a series of operations are performed on the computer or other programmable device to produce a computer implemented process. As a result, the instructions executed on the computer or other programmable device provide steps for performing the functions specified in one or more processes in the flowchart or one or more blocks in the block diagram.

In the description of this specification, reference terms such as "one embodiment", "one specific embodiment", "some embodiments", "for example", "example", "specific example", or "some examples" are intended to mean that the specific features, structures, materials, or characteristics described in connection with that embodiment or example are included in at least one embodiment or example of the present disclosure. In this specification, the illustrative expressions of the above terms do not necessarily refer to the same embodiment or example. Furthermore, the described specific features, structures, materials, or characteristics may be combined in any suitable manner in one or more embodiments or examples.

The specific embodiments described above provide a further detailed illustration of the objectives, technical solutions, and beneficial effects of the present disclosure. It should be understood that the above description is merely specific embodiments of the present disclosure and is not intended to limit the scope of protection of the disclosure. Any modifications, equivalent substitutions, improvements, etc., made within the spirit and principles of the present disclosure should be included within the scope of protection of the present disclosure.

## Claims

1. A method for predicting chemical states and a chemical reaction in a multiphase system, comprising:
obtaining a key condition corresponding to the multiphase system, wherein the key condition comprises: temperature, pressure, a plurality of element types, and respective amounts thereof;
predicting and obtaining chemical state information under the key condition by using a Gibbs free energy minimization method; wherein the chemical state information comprises a plurality of pure substance species and respective amounts thereof when the multiphase system reaches thermodynamic equilibrium; and
predicting and obtaining a chemical reaction that will occur in the multiphase system under the key condition based on the chemical state information.

2. The method for predicting the chemical states and the chemical reaction in the multiphase system according to claim 1, wherein a plurality of key conditions are provided, a key influencing parameter of the multiphase system include at least one selected from a impurity element type among the plurality of element types, temperature, and pressure, values of the key influencing parameter corresponding to the respective key conditions are different;
correspondingly, after obtaining the key condition corresponding to the multiphase system, the method further comprises:
predicting and obtaining the chemical state information under each key condition by using a Gibbs free energy minimization method;
predicting and obtaining the chemical reaction that will occur in the multiphase system under each key condition based on the chemical state information under each key condition.

3. The method for predicting the chemical states and the chemical reaction in the multiphase system according to claim 2, wherein after predicting and obtaining the chemical state information under each key condition using the Gibbs free energy minimization method, the method further comprises:
selecting, from the chemical state information under each key condition, the pure substance species containing a preset selected element and respective amounts thereof;
generating a two-dimensional projected phase diagram corresponding to the preset selected element according to the pure substance species containing the preset selected element and respective amounts thereof;
wherein the two-dimensional projected phase diagram is divided into a plurality of regions, each region corresponds to a group of pure substances, and the groups of the pure substances corresponding to the respective regions are different, each group of the pure substances is obtained according to the pure substance species containing the preset selected element, a value of a phase boundary line between the respective regions is a preset phase boundary line value, the two-dimensional projected phase diagram further includes: variation curves of each pure substance species containing the selected element.

4. The method for predicting the chemical states and the chemical reaction in the multiphase system according to claim 3, wherein selecting, from the chemical state information under each key condition, the pure substance species containing the preset selected element and the respective amounts thereof, comprises:
performing interpolation processing based on the respective key conditions and chemical state information corresponding thereto to obtain interpolated key conditions and chemical state information corresponding respectively thereto;
selecting, from the chemical state information under each interpolated key condition, the pure substance species containing the preset selected element and the respective amounts thereof.

5. A prediction apparatus for the chemical states and a chemical reaction in a multiphase system, comprising:
obtainment module configured to obtain a key condition corresponding to the multiphase system, wherein the key condition includes: temperature, pressure, a plurality of element types, and respective amounts thereof;
first calculation module configured to predict and obtain chemical state information under the key condition by using a Gibbs free energy minimization method, wherein the chemical state information comprises a plurality of pure substance species and respective amounts thereof when the multiphase system reaches thermodynamic equilibrium;
first prediction module configured to predict and obtain a chemical reaction that will occur in the multiphase system under the key condition based on the chemical state information.

6. The prediction apparatus for the chemical states and the chemical reaction in the multiphase system according to claim 5, wherein a plurality of key conditions are provided, a key influencing parameter of the multiphase system include at least one selected from a impurity element type among the plurality of element types, temperature, and pressure, values of the key influencing parameter corresponding to the respective key conditions are different;
correspondingly, the prediction apparatus for the chemical state and the chemical reaction in the multiphase system further comprises:
second calculation module configured to predict and obtain the chemical state information under each key condition by using a Gibbs free energy minimization method;
second prediction module configured to predict and obtain the chemical reaction that will occur in the multiphase system under each key condition based on the chemical state information under each key condition.

7. The prediction apparatus for the chemical states and the chemical reaction in the multiphase system according to claim 6, wherein the apparatus further comprises:
extraction module configured to select, from the chemical state information under each key condition, pure substance species containing a preset selected element and respective amounts thereof;
phase diagram plotting module configured to generate a two-dimensional projected phase diagram corresponding to the preset selected element according to the pure substance species containing the preset selected element and the respective amounts thereof;
wherein the two-dimensional projected phase diagram is divided into a plurality of regions, each region corresponds to a group of pure substances, and the groups of the pure substances corresponding to the respective regions are different, each group of the pure substances is obtained according to the pure substance species containing the preset selected element, a value of a phase boundary line between the respective regions is a preset phase boundary line value, the two-dimensional projected phase diagram further includes: variation curves of each pure substance species containing the selected element.

8. The prediction apparatus for the chemical states and the chemical reaction in the multiphase system according to claim 7, wherein the extraction module comprises:
interpolation processing unit configured to perform interpolation processing based on the respective key conditions and chemical state information corresponding thereto to obtain the interpolated key conditions and chemical state information corresponding respectively thereto;
extraction unit configured to select, from the chemical state information under each interpolated key condition, the pure substance species containing the preset selected element and the respective amounts thereof.

9. An electronic device, comprising a memory, a processor, and a computer program stored in the memory and executable on the processor, wherein when executing the program, the processor implements the method for predicting the chemical states and the chemical reaction in the multiphase system according to any one of claims 1 to 4.

10. A computer readable storage medium, wherein the computer readable storage medium stores a computer instruction which, when executed by a processor, implements the method for predicting the chemical states and the chemical reaction in the multiphase system according to any one of claims 1 to 4.
